# EUROPÄISCHE PATENTSCHRIFT

(11) **EP 1 758 560 B1**
(45) Veröffentlichungstag und Bekanntmachung des Hinweises auf die Patenterteilung: **29.06.2016**
(21) Anmeldenummer: 05743447.4
(22) Anmeldetag: 27.05.2005
(51) Int. Cl.: A61K 9/36, A61K 31/565, A61K 31/57

(54) **HORMONALE ZUBEREITUNG ENTHALTEND EINE KOMBINATION AUS ETHINYLESTRADIOL UND CHLORMADINONACETAT**
HORMONAL COMPOSITION CONTAINING A COMBINATION OF ETHINYL ESTRADIOL AND CHLORMADINONE ACETATE
COMPOSITION HORMONALE CONTENANT UNE ASSOCIATION D'ETHINYLESTRADIOL ET D'ACETATE DE CHLORMADINONE

(30) Priorität: 28.05.2004 DE 102004026679
(43) Veröffentlichungstag der Anmeldung: 07.03.2007
(73) Patentinhaber: Richter Gedeon Nyrt., 1103 Budapest (HU)
(72) Erfinder: SCHRAMM, Georg, 52224 Stolberg (DE); HENSKE, Rainer, 45355 Essen (DE)
(74) Vertreter: Kutzenberger, Helga
(86) Internationale Anmeldenummer: PCT/EP2005/005762
(87) Internationale Veröffentlichungsnummer: WO 2005/115350

(56) Entgegenhaltungen:
- DE-A1- 4 321 957
- US-B1- 6 451 779
- US-B1- 6 500 814
- WINKLER U H ET AL: "A COMPARATIVE STUDY OF THE HEMOSTATIC EFFECTS OF TWO MONOPHASIC ORAL CONTRACEPTIVES CONTAINING 30 MUG ETHINYLESTRADIOL AND EITHER 2 MG CHLORMADIONE ACETATE OR 150 MUG DESOGESTREL" EUROPEAN JOURNAL OF CONTRACEPTION AND REPRODUCTIVE HEALTH CARE, PARTHENON PUBLISHING GROUP, CARNFOORTH, GB, Bd. 4, Nr. 3, September 1999 (1999-09), Seiten 145-154, XP009052167 ISSN: 1362-5187
- RAUDRANT D ET AL: "PROGESTOGENS WITH ANTIANDROGENIC PROPERTIES" DRUGS, ADIS INTERNATIONAL LTD, AT, Bd. 63, Nr. 5, 2003, Seiten 463-492, XP008038646 ISSN: 0012-6667

## Beschreibung

Die vorliegende Erfindung betrifft die Verwendung einer Kombination bestehend aus 5 bis 50 µg Ethinylestradiol und 1 bis 5 mg Chlormadinonacetat zur Herstellung eines Arzneimittels zur oralen, monophasigen Verabreichung von Tages-Einheiten für die Dauer von mindestens 120 Tagen und gegebenenfalls zur anschließenden 3- bis 7-tägigen Verabreichung hormonfreier Tages-Einheiten zur Behandlung von Dysmenorrhoe und/oder von menstruationszyklusabhängigen Beschwerden, wie dem prämenstruellen Syndrom, wie Kopfschmerzen/Migräne und/oder menstruationszyklus-beeinflussten Erkrankungen, wie Depression, und/oder zur Reduktion der Anzahl von Blutungen, wie Entzugsblutungen und/oder Zwischenblutungen.

An hormonale Kontrazeptiva wird von Frauen immer öfter die Anforderung gestellt, dass möglichst viele Blutungen, wie Entzugsblutungen oder Zwischenblutungen ausbleiben, da mit dem Ausbleiben von Blutungen weniger menstruelle Beschwerden, eine besserer Hygiene, eine höherer Lebensqualität und weniger Blutverlust bzw. Eisenmangel gewährleistet sind.

Aus DE 698 04 918 ist bereits ein niedrig dosiertes, orales Kontrazeptivum umfassend eine monophasige Verabreichung von Tages-Einheiten mit einer Kombination aus Östrogen und Progestin über 60 bis 110 aufeinanderfolgenden Tagen, gefolgt von einer 3 bis 7-tägigen Einnahmepause bekannt, wobei die tägliche Menge Östrogen bzw. Gestagen zu 5 bis 35 µg Ethinylestradiol bzw. 0,025 bis 10 mg Norethisteronacetat äquivalent ist.

Darüber hinaus besteht ein weiterer Bedarf, die Anzahl von Blutungen, wie Entzugsblutungen und/oder Zwischenblutungen, weiter zu reduzieren, um eine Verbesserung der Lebensqualität von Frauen zu erzielen sowie u. a. androgenabhängige Beschwerden bzw. Erkrankungen anhaltend zu therapieren.

Daher war es die Aufgabe der vorliegenden Erfindung, ein hormonales Arzneimittel zur Verfügung zu stellen, das u. a. eine gegenüber dem Stand der Technik wesentliche Reduktion der Anzahl von Blutungen, insbesondere von Entzugsblutungen und/oder Zwischenblutungen, ermöglicht.

Diese Aufgabe wird durch das zur Verfügung stellen des erfindungsgemäßen hormonalen Arzneimittels zur ununterbrochenen, oralen Verabreichung an Frauen gelöst, das dadurch gekennzeichnet ist, dass es aus mindestens 120 hormonhaltigen Tages-Einheiten, die 5 bis 50 µg Ethinylestradiol und 1 bis 5 mg Chlormadinonacetat enthalten, und gegebenenfalls aus 7 bis 3 hormonfreien Tages-Einheiten besteht.

In einer erfindungsgemäßen Ausführungsform kann das hormonale Arzneimittel hormonhaltige Tages-Einheiten für eine ununterbrochene Verabreichung für die Dauer von 120 Tagen bis zu mehreren Jahren, vorzugsweise bis zu 2 Jahren, besonders bevorzugt bis zu einem Jahr, vorsehen, gegebenenfalls gefolgt von 7 bis 3 hormonfreien Tages-Einheiten.

Durch die monophasige Einnahme des erfindungsgemäßen, hormonalen Langzeit-Arzneimittels wird eine gegenüber dem Stand der Technik wesentliche Reduktion der Anzahl von Blutungen, insbesondere der Entzugsblutungen und/oder Zwischenblutungen, erreicht.

Darüber hinaus kann die ununterbrochene Einnahme des hormonalen Arzneimittels auch aus therapeutischen Gründen erfolgen, wie z. B. zur Behandlung von Dysmenorrhoe und /oder bei menstruationszyklusabhängigen Beschwerden, wie dem prämenstruellen Syndrom, insbesondere von Kopfschmerzen/Migräne, und/oder von menstruationszyklusbeeinflussten Erkrankungen, wie Depression.

Weiterhin kann durch die Einnahme des erfindungsgemäßen hormonalen Arzneimittels auch eine Linderung typischer Symptome in der Prä- und Perimenopause bewirkt werden. Daher ist das erfindungsgemäße hormonale Arzneimittel insbesondere auch für Frauen mit einem Alter über 35 Jahre, also Frauen in Prä- und Perimenopause, besonders geeignet.

Typische Symptome in der Prä- und Perimenopause sind beispielsweise ein unregelmäßiger Menstruationszyklus, vasomotorische Störungen, wie Hitzewallungen, Schweißausbrüche und/oder Schlaflosigkeit. Des weiteren leiden auch viele Frauen in der Prä- und Perimenopause an den vorstehend aufgeführten androgenabhängigen Symptomen. Insbesondere in der Altersklasse über 35 Jahre kann es bedingt durch Hormonschwankungen zur Bildung von Damenbart, einer tiefen Stimme, Hautunreinheiten und/oder Haarausfall kommen.

Unter anderem kann daher durch die Einnahme des erfindungsgemäßen hormonalen Arzneimittels über mindestens 120 Tage zumindest eine Linderung der androgenabhängigen Symptome in der Prä- und Perimenopause erreicht werden.

Ein weiterer Gegenstand der vorliegenden Erfindung ist daher auch die Verwendung einer Kombination von Ethinylestradiol und Chlormadinonacetat zur Herstellung eines Arzneimittels zur oralen monophasigen, ununterbrochenen Verabreichung von hormonhaltigen Tages-Einheiten an Frauen für die Dauer von mindestens 120 Tagen, vorzugsweise von 120 Tagen bis mehreren Jahren, besonders bevorzugt bis zu 2 Jahren, ganz besonders bevorzugt bis zu einem Jahr und gegebenenfalls 3 bis 7 aufeinanderfolgenden, hormonfreien Tages-Einheiten zur Behandlung von Dysmenorrhoe, von menstruationszyklusabhängige Beschwerden, wie dem prämenstruellen Syndrom, wie Kopfschmerzen/Migräne, und/oder menstruationszyklusbeeinflusste Erkrankungen, wie Depression, und/oder zur Reduktion der Anzahl von Blutungen, wie Entzugsblutungen und/oder Zwischenblutungen.

Das erfindungsgemäße hormonale Arzneimittel enthält Ethinylestradiol als Östrogen und Chlormadinonacetat als Gestagen. Durch die Einnahme dieser Kombination aus Ethinylestradiol und Chlormadinonacetat über mindestens 120 Tage wird mit dem erfindungsgemäßen Arzneimittel eine gegenüber dem Stand der Technik wesentliche Reduktion der Anzahl von Blutungen, sowohl Entzugsblutungen als auch Zwischenblutungen, und ein verbessertes Wohlbefinden erzielt.

Außerdem ist die therapeutische Behandlung der vorstehend aufgeführten Beschwerden bzw. Erkrankungen möglich.

Jede hormonale Tages-Einheit des erfindungsgemäßen hormonalen Arzneimittels enthält vorzugsweise 1 bis 5 mg Chlormadinonacetat und 5 bis 50 µg Ethinylestradiol, bevorzugt 1 bis 3 mg Chlormadinonacetat und 15 bis 30 µg Ethinylestradiol, besonders bevorzugt 1 bis 2 mg Chlormadinonacetat und 20 bis 30 µg Ethinylestradiol.

Gemäß einer erfindungsgemäßen Ausführungsform kann das erfindungsgemäße Arzneimittel 15 µg, 20 µg oder 30 µg Ethinylestradiol und 1 mg, 2 mg, 3 mg, 4 mg oder 5 mg Chlormadinonacetat enthalten.

Alle hormonhaltigen Tageseinheiten des erfindungsgemäßen, monophasigen Arzneimittels enthalten vorzugsweise jeweils dieselbe Menge an Chlormadinonacetat. Dies gilt auch für die Menge an Ethinylestradiol.

Die Zahl der Blutungen, wie Entzugsblutungen und/oder Zwischenblutungen, wird durch die Einnahme des erfindungsgemäßen Arzneimittels für die Dauer von mindestens 120 Tagen bis zu mehreren Jahren reduziert, wenn die Tages-Einheiten vorzugsweise von 1 bis 5 mg, besonders bevorzugt 1 bis 3 mg Chlormadinonacetat und von 5 bis 50 µg, besonders bevorzugt 20 bis 30 µg Ethinylestradiol enthalten.

Vorzugsweise enthält jede Tages-Einheit mengenmäßig dieselbe Hormonkombination.

An die ununterbrochene Einnahmephase über die Dauer von mindestens 120 Tagen bis zu mehreren Jahren, vorzugsweise bis zu 2 Jahren, ganz besonders bevorzugt bis zu einem Jahr von hormonhaltigen Tages-Einheiten kann sich eine Einnahmepause von 7 bis 3 Tagen oder eine Einnahme von 7 bis 3 hormonfreien Tages-Einheiten anschließen, bevor mit der Einnahme eines weiteren erfindungsgemäßen hormonalen Arzneimittels angefangen wird.

Das erfindungsgemäße Arzneimittel kann auch als ein Kit umfassend mehrere erfindungsgemäße Arzneimittel-Blister jeweils zur ununterbrochenen Verabreichung von mindestens 120 hormonhaltigen Tages-Einheiten, die jeweils 5 bis 50 µg Ethinylestradiol und 1 bis 5 mg Chlormadinonacetat enthalten, und gegebenenfalls 7 bis 3 hormonfreien Tagen-Einheiten, wobei die ununterbrochene Verabreichung des nächsten Arzneimittel-Blisters aus dem Kit unmittelbar an die Verabreichung der hormonfreien Tages-Einheiten bzw. an eine entsprechend lange Einnahmepause anschließt. Vorzugsweise enthalten alle hormonhaltigen Tages-Einheiten mengenmäßig dieselbe Hormonkombination.

Die Tages-Einheiten des erfindungsgemäßen hormonalen Arzneimittels können vorzugsweise in der Form von Tabletten vorliegen. Herstellungsmethoden für solche Tages-Einheiten sind dem Fachmann bekannt. Als Zusatzstoffen neben der Kombination aus Chlormadinonacetat und Ethinylestradiol können gegebenenfalls bekannte Hilfsstoffe mitverwendet werden. Vorzugsweise werden die Tabletten als Tages-Einheiten in Blister verpackt und für die tägliche Einnahme gekennzeichnet.

### Beispiele

### Beispiel 1:

**Zusammensetzung**

| | Pro Tablette |
|---|---|
| Ethinylestradiol | 0,020 mg |
| Chlormadinonacetat | 2,000 mg |
| Povidon K30 | 3,000 mg |
| Lactose | 31,980 mg |
| Maisstärke | 12,000 mg |
| Magnesiumstearat | 0,500 mg |
| Hochdisperses Siliciumdioxid | 0,500 mg |

Ethinylestradiol (EE) und Povidone K 30 (Polyvinylpyrrolidon) wurden in 600 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.

Die Tabletten wurden mit einem Lack auf Basis Methylhydroxypropylcellulose überzogen (z. B. Opadry YS-1-2184 vom Hersteller Colorcon); Überzugsmasse 2 mg pro Tablette und als Arzneimittel mit 120 Tages-Einheiten in einem Blister verpackt.

### Beispiel 2:

**Zusammensetzung**

| | Pro Tablette |
|---|---|
| Ethinylestradiol | 0,03 mg |
| Chlormadinonacetat | 2,000 mg |
| Povidon K30 | 3,000 mg |
| Lactose | 31,970 mg |
| Maisstärke | 12,000 mg |
| Magnesiumstearat | 0,500 mg |
| hochdisperses Siliciumdioxid | 0,500 mg |

Ethinylestradiol (EE) und Povidone K 30 (PVP) wurden in 600 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepreßt.

Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa·s, | 0,1351 kg |
| Polyethylenglykol 6000 | 0,0395 kg |
| Propylenglykol | 0,0054 kg |
| Gereinigtes Wasser | 1,6200 kg |

Diese Tabletten wurden jeweils zu einem Arzneimittel mit 189 Tages-Einheiten in einem Blister verpackt und zu einem Kit mit mehreren Arzneimittel-Blistern zusammengefasst, wobei zwischen 2 Verabreichungszyklen von jeweils 189 Tages-Einheiten jeweils eine Einnahmepause von 7 Tagen vorgesehen ist.

### Beispiel 3:

**Zusammensetzung**

| | Pro Tablette |
|---|---|
| Ethinylestradiol | 0,015 mg |
| Chlormadinonacetat | 2,000 mg |
| Povidon K30 | 4,000 mg |
| Lactose | 63,485 mg |
| Maisstärke | 10,000 mg |
| Magnesiumstearat | 0,500 mg |

Ethinylestradiol (EE) und Povidone K 30 (PVP) wurden in 950 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat gemischt und auf einer Tablettenpresse mit 6 mm Stempeln zu Tabletten mit einem Gewicht von 80 mg gepresst.

Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa·s, | 0,1351 kg |
| Polyethylenglykol 6000 | 0,0395 kg |
| Propylenglykol | 0,0054 kg |
| Gereinigtes Wasser | 1,6200 kg |

Die überzogenen Tabletten wurden jeweils zu einem Arzneimittel mit 365 Tages-Einheiten verpackt. Ein Kit umfassend mehrere Arzneimittel-Blister sieht jeweils eine Einnahmepause von 7 Tagen vor.

### Beispiel 4:

**Zusammensetzung**

| | Pro Tablette |
|---|---|
| Ethinylestradiol | 0,030 mg |
| Chlormadinonacetat | 5,000 mg |
| Povidon K30 | 4,500 mg |
| Lactose | 60,470 mg |
| Maisstärke | 10,000 mg |
| Magnesiumstearat | 0,500 mg |

Ethinylestradiol (EE) und Povidone K 30 (PVP) wurden in 950 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat gemischt und auf einer Tablettenpresse mit 6 mm Stempeln zu Tabletten mit einem Gewicht von 80 mg gepresst.

Die Tabletten wurden mit einem Lack auf Basis Methylhydroxypropylcellulose mit folgender Zusammensetzung überzogen (Überzugsmasse 1 mg pro Tablette)

| | |
|---|---|
| Methylhydroxypropylcellulose 6 mPa·s, | 0,068 kg |
| Polyethylenglykol 6000 | 0,020 kg |
| Propylenglykol | 0,002 kg |
| Gereinigtes Wasser | 0,810 kg |

Als Arzneimittel wurden die überzogenen Tabletten als 150 Tages-Einheiten zur ununterbrochenen Verabreichung in einem Blister verpackt. Ein Kit aus mehreren Arzneimittel-Blistern sieht zwischen 2 Einnahmezyklen jeweils eine Einnahmepause von 4 Tagen vor.

## Patentansprüche

1. Verwendung einer Kombination bestehend aus 5 bis 50 µg Ethinylestradiol und 1 bis 5 mg Chlormadinonacetat zur Herstellung eines Arzneimittels zur oralen, monophasigen Verabreichung von Tages-Einheiten für die Dauer von mindestens 120 Tagen und gegebenenfalls zur anschließenden 3- bis 7-tägigen Verabreichung hormonfreier Tages-Einheiten zur Behandlung von Dysmenorrhoe und/oder von menstruationszyklusabhängigen Beschwerden, wie dem prämenstruellen Syndrom, wie Kopfschmerzen/Migräne, und/oder von menstruationszyklus-beeinflussten Erkrankungen, wie Depression, und/oder zur Reduktion der Anzahl von Blutungen, wie Entzugsblutungen und/oder Zwischenblutungen.

2. Verwendung nach Anspruch 1, **dadurch gekennzeichnet, dass** die Kombination aus 20 bis 30 µg Ethinylestradiol und 1 bis 3 mg Chlormadinonacetat besteht.

3. Verwendung nach Anspruch 1 oder 2, **dadurch gekennzeichnet, dass** alle hormonhaltigen Tages-Einheiten mengenmäßig dieselbe Hormonkombination enthalten.

4. Verwendung nach einem der Ansprüche 1 bis 3, **dadurch gekennzeichnet, dass** die hormonhaltigen Tages-Einheiten jeweils dieselbe Menge Ethinylestradiol bzw. jeweils dieselbe Menge Chlormadinonacetat aufweisen.

5. Verwendung nach einem der Ansprüche 1 bis 4, **dadurch gekennzeichnet, dass** die Tages-Einheiten als Tabletten vorliegen.

6. Verwendung nach Anspruch 5, **dadurch gekennzeichnet, dass** die Tabletten neben der Kombination aus Chlormadinonacetat und Ethinylestradiol aus bekannten Hilfsstoffen hergestellt sind.

7. Verwendung nach einem der Ansprüche 1 bis 6, **dadurch gekennzeichnet, dass** die hormonhaltigen Tages-Einheiten für eine ununterbrochene Verabreichung für die Dauer von 120 Tagen bis mehreren Jahren, vorzugsweise bis 2 Jahren, besonders bevorzugt bis zu einem Jahr und gegebenenfalls hormonfreie Tages-Einheiten für eine 3 bis 7-tägige Verabreichung reichen.

8. Verwendung nach einem der Ansprüche 1 bis 7 zur Behandlung von Frauen in der Prä- oder Perimenopause.

## Claims

1. Use of a combination containing 5-50µg of ethinyloestradiol and 1-5mg of chlormadinone acetate for preparation of medicament for oral mono-phase administration of daily units for a duration of at least 120 days uninterruptedly, which is optionally followed by administration of hormone-free daily units on the subsequent 3 to 7 days for the treatment of dysmenorrhoea and/or conditions influenced by the menstrual cycle such as premenstrual syndrome, such as headache/migraine, and/or illnesses influenced by the menstrual cycle such as depression, and/or reduction in the instances of bleedings such as withdrawal bleedings and/or intermenstrual bleedings.

2. Use according to claim 1 wherein said combination contains 20 to 30µg of ethinyloestradiol and 1 to 3mg chlormadinone acetate.

3. Use according to claim 1 or 2 wherein all hormone containing daily units comprises the same combination of hormones quantitatively.

4. Use according to claims 1 to 3, wherein each of the hormone containing daily units comprises the same quantity of ethinyloestradiol and the same quantity of chlormadinone acetate, respectively.

5. Use according to claims 1 to 4, wherein the daily units are in form of tablets.

6. Use according to claim 5, wherein besides the combination of chlormadinone acetate and ethinyloestradiol each tablet is prepared from known excipients.

7. Use according to claims 1 to 6, wherein the hormone-containing daily units are enough for an administration over an uninterrupted period of 120 days to several years, preferably up to two years, particularly preferable up to one year, and optionally the hormone-free daily units for an administration over 3 to 7 days.

8. Use according to claims 1 to 7 for the treatment of pre- and perimenopausal women.

## Revendications

1. Utilisation d'une association consistant en 5 à 50 µg d'éthynylestradiol et 1 à 5 mg d'acétate de chlormadinone pour fabriquer un médicament destiné à une administration orale monophasée d'unités journalières pendant une durée d'au moins 120 jours, et éventuellement pour une administration ultérieure de 3 à 7 jours d'unités journalières sans hormones pour le traitement de la dysménorrhée et/ou des symptômes dépendant du cycle menstruel tels que le syndrome prémenstruel, comme les céphalées/les migraines, et/ou de maladies influencées par le cycle menstruel, comme la dépression, et/ou pour la réduction du nombre d'hémorragies telles que les hémorragies de privation et/ou les hémorragies du milieu de cycle.

2. Utilisation selon la revendication 1, **caractérisée en ce que** l'association est constituée de 20 à 30 µg d'éthynylestradiol et de 1 à 3 mg d'acétate de chlormadinone.

3. Utilisation selon la revendication 1 ou 2, **caractérisée en ce que** toutes les unités journalières contenant des hormones contiennent la même association d'hormones, en quantité.

4. Utilisation selon l'une des revendications 1 à 3, **caractérisée en ce que** chacune des unités journalières contenant des hormones présentent la même quantité d'éthynylestradiol, ou la même quantité d'acétate de chlormadinone.

5. Utilisation selon l'une des revendications 1 à 4, **caractérisée en ce que** les unités journalières se présentent sous forme de comprimés.

6. Utilisation selon la revendication 5, **caractérisée en ce que** les comprimés sont fabriqués, en plus de l'association d'acétate de chlormadinone et d'éthynylestradiol, à partir d'adjuvants connus.

7. Utilisation selon l'une des revendications 1 à 6, **caractérisée en ce que** les unités journalières contenant des hormones suffisent pour une administration ininterrompue sur une durée de 120 jours à plusieurs années, de préférence jusqu'à 2 ans, d'une manière particulièrement préférée jusqu'à 1 an, et éventuellement les unités journalières sans hormones suffisent à une administration de 3 à 7 jours.

8. Utilisation selon l'une des revendications 1 à 7 pour le traitement de femmes en pré- ou périménopause.
